**Europäisches Patentamt**

**European Patent Office**

**Office européen des brevets**

(19)

(11) Veröffentlichungsnummer: **0 238 050**
**B1**

(12)

# EUROPÄISCHE PATENTSCHRIFT

(45) Veröffentlichungstag der Patentschrift:
**04.10.89**

(51) Int. Cl.⁴: **C08F 2/10, A61L 15/00**

(21) Anmeldenummer: **87103895.6**

(22) Anmeldetag: **17.03.87**

(54) **Verfahren zur diskontinuierlichen Herstellung von vernetzten, feinteiligen Polymerisaten.**

(30) Priorität: **21.03.86 DE 3609545**

(43) Veröffentlichungstag der Anmeldung:
**23.09.87 Patentblatt 87/39**

(45) Bekanntmachung des Hinweises auf die Patenterteilung:
**04.10.89 Patentblatt 89/40**

(84) Benannte Vertragsstaaten:
**AT BE CH DE FR GB IT LI NL SE**

(56) Entgegenhaltungen:
**DE-A- 3 233 778**
**US-A- 4 255 545**

(73) Patentinhaber: **BASF Aktiengesellschaft,
Carl-Bosch-Strasse 38, D-6700 Ludwigshafen(DE)**

(72) Erfinder: **Nowakowsky, Bernhard Henryk, Dr.,
Schuckertstrasse 10, D-6700 Ludwigshafen(DE)**
Erfinder: **Beck, Juergen, Siegfriedstrasse 31,
D-6800 Mannheim(DE)**
Erfinder: **Hartmann, Heinrich, Dr., Weinheimer
Strasse 46, D-6703 Limburgerhof(DE)**
Erfinder: **Vamvakaris, Christos, Dr., Riedweg 6,
D-6701 Kallstadt(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der DE-OS 34 32 690 ist ein Verfahren zur kontinuierlichen Herstellung von vernetzten Polymerisaten bekannt, bei dem man wasserlösliche Monomere in Gegenwart eines Vernetzers und von Initiatoren in einem Kessel polymerisiert, der mit einer Mehrzahl von parallel zueinander angeordneten rotierenden Rührwellen ausgerüstet ist, die mit Rührerblättern versehen sind. Die Polymerisation wird kontinuierlich in einem Zweiarm-Typkneter oder beispielsweise in einem Dreischaft-Kneter durchgeführt. Die Polymerisationstemperatur liegt vorzugsweise in dem Bereich von 70 bis 100°C. Bei diesem Reaktortyp findet eine starke Rückvermischung statt, so daß die Monomerlösung auf das feinverteilte wasserhaltige Gelpolymere gegeben wird und die Polymerisation des Monomeren auf der Oberfläche des Polymergels abläuft. Die so herstellbaren feinteiligen Polymerisatgele haben einen relativ hohen Restmonomergehalt und enthalten erhebliche Mengen an extrahierbaren, d.h. löslichen Anteilen. Sie müssen daher in einem gesonderten Verfahrensschritt einer Nachpolymerisation und Nachvernetzung unterworfen werden.

Der Erfindung liegt die Aufgabe zugrunde, ein Verfahren zur Herstellung von vernetzten, feinteiligen, wasserabsorbierenden Polymerisaten zur Verfügung zu stellen, bei dem man Polymerisate mit einem geringen Restmonomerengehalt sowie geringen Mengen an extrahierbaren Anteilen erhält.

Die Aufgabe wird erfindungsgemäß gelöst mit einem Verfahren zur diskontinuierlichen Herstellung von vernetzten, feinteiligen, wasserabsorbierenden Polymerisaten durch Copolymerisieren von 100 Gew.-Teilen eines Monomeren aus der Gruppe a) oder eines Monomerengemische aus den Gruppen

a) 50 bis 100 Gew.-Teilen zu jeweils 0 bis 100 Mol% mit Alkalimetall- oder Ammoniumbasen neutralisierter Acrylsäure oder Methacrylsäure, Acrylamid, Methacrylamid und N-Vinylpyrrolidon,
b) 0 bis 30 Gew.-Teilen anderen wasserlöslichen monoethylenisch ungesättigten Monomeren und
c) 0 bis 20 Gew.-Teilen wasserunlöslichen monoethylenisch ungesättigten Monomeren

mit 0,01 bis 5 Gew.-Teilen eines mindestens zwei ethylenisch ungesättigte Doppelbindungen enthaltenden Monomeren als Vernetzer in 20 bis 80 gew.%iger wäßriger Lösung in Gegenwart von Initiatoren bei Temperaturen oberhalb von 45°C, wenn man die Copolymerisation mehrstufig in einer diskontinuierlich arbeitenden Mischvorrichtung unter ständiger Durchmischung in allen Stufen durchführt, wobei man

in der ersten Stufe die wäßrige Monomerlösung bei Temperaturen von 45 bis 95°C und einem Druck von 0,1 bis 0,8 bar unter partiellem Abdestillieren von Wasser copolymerisiert,
in der zweiten Stufe die Copolymerisation bei Temperaturen von 100 bis 170°C unter einem Druck bis zu 8 bar vervollständigt und nach dem Entspannen

in der dritten Stufe den Wassergehalt des so hergestellten feinteiligen Copolymerisats unter vermindertem Druck bei 70 bis 180°C oder unter Atmosphärendruck bei 120 bis 180°C auf 0,5 bis 10 Gew.% erniedrigt. Die Copolymerisation wird vorzugsweise in einem diskontinuierlich arbeitenden Kneter mit einem Selbstreinigungseffekt von mindestens 80 % durchgeführt.

Wasserabsorbierende Copolymerisate auf Basis von Polymerisaten der Acrylsäure, Methacrylsäure und deren Amiden sowie von N-Vinylpyrrolidon werden durch Copolymerisieren der Monomeren zusammen mit einem Vernetzer hergestellt. Als Monomere der Gruppe a) kommen Acrylsäure und/oder Methacrylsäure in Betracht, die jeweils zu 0 bis 100 Mol% mit Alkalimetall- oder Ammoniumbasen neutralisiert sind. Zu dieser Gruppe von Monomeren gehören außerdem Acrylamid, Methacrylamid und N-Vinylpyrrolidon. Für die partielle bzw. vollständige Neutralisation der Acrylsäure bzw. Methacrylsäure verwendet man vorzugsweise Natronlauge und/oder Kalilauge. Die Neutralisation kann selbstverständlich auch mit Hilfe von Soda oder Pottasche sowie mit Ammoniak oder substituierten Aminen, wie Trimethylamin, Tri-n-octylamin und Triethanolamin vorgenommen werden. Sofern man Acrylsäure oder Methacrylsäure zur Polymerisation einsetzt, kann die Neutralisation bereits vor der Polymerisation, während der Polymerisation oder auch im Anschluß an die Polymerisation vorgenommen werden. Die Monomeren der Gruppe a) können entweder allein oder in Mischung untereinander in jedem beliebigen Verhältnis bei der Copolymerisation mit den Vernetzern eingesetzt werden. So kann man beispielsweise Acrylsäure allein oder Mischungen aus Acrylsäure und Methacrylsäure bzw. aus Acrylsäure und Acrylamid oder aus Acrylsäure, Acrylamid und Methacrylamid oder auch Mischungen aus Acrylamid und N-Vinylpyrrolidon der Copolymerisation unterwerfen. Vorzugsweise verwendet man jedoch als Monomer der Gruppe a) zu 50 bis 100 Mol% mit Natronlauge und/oder Kalilauge neutralisierte Acrylsäure. Die Neutralisation der Acrylsäure wird dabei vorzugsweise vor der Polymerisation oder während der ersten Stufe der Polymerisation durchgeführt. Die Monomeren der Gruppe a) können entweder allein oder in Mischung mit den Monomeren der Gruppen b) und c) mit den Vernetzern copolymerisiert werden. Im Fall von Monomerenmischungen aus den Gruppen a) bis c) beträgt der Anteil der Monomeren der Gruppe a) 50 bis 99 %.

Die Monomeren der Gruppe b) bestehen aus von den Monomeren der Gruppe a) verschiedenen, anderen wasserlöslichen, monoethylenisch ungesättigten Monomeren. Hierzu gehören beispielsweise Maleinsäure, Fumarsäure, Crotonsäure, Itaconsäure, Vinylsulfonsäure, Vinylpyridiniumsalze, N-Vinylformamid, basische Acrylate oder Methacrylate in Form der Salze mit starken Mineralsäuren oder in quaternisierter Form, z.B. Dimethylaminoethylacrylat, Diethylaminoethylacrylat, Dimethylaminopropylacrylat, Dimethylaminobutylacrylat, Diethylaminoethylmethacrylat, Dimethylaminoethylmethacrylat und Dimethylaminopropylacrylat. Zu dieser Gruppe von Monomeren gehören auch die Hydroxialkylacrylate und Hydroxialkylmethacrylate, z.B. Hydroxiethylacrylat, Hydroxiethylmethacrylat, Hydroxipropylacrylate, Hydroxipropylmethacrylate, Hydroxibutylacrylate, Hydroxibutylmethacrylate sowie Acrylsäureester und Methacrylsäureester, die durch Veresterung von Polyethylenglykolen mit Acrylsäure bzw. Methacrylsäure im Molverhältnis 1:1 erhalten werden. Die für die Veresterung eingesetzten Polyethylenglykole haben Molekulargewichte von 126 bis 8500. Pro 100 Gew.-Teile der Monomeren der Gruppe a) verwendet man 0 bis 30, vorzugsweise 0,5 bis 15 Gew.-Teile der Monomeren der Gruppe b).

Zu den Monomeren der Gruppe c) gehören wasserunlösliche monoethylenisch ungesättigte Monomere. Hierbei handelt es sich beispielsweise um die Ester der Acrylsäure oder Methacrylsäure mit einwertigen, 1 bis 18 Kohlenstoffatome enthaltenden einwertigen Alkoholen, z.B. Methylacrylat, Ethylacrylat, Propylacrylat, Isopropylacrylat, n-Butylacrylat, Isobutylacrylat, Hexylacrylat, 2-Ethylhexylacrylat, Stearylacrylat, die entsprechenden Ester der Methacrylsäure, Fumarsäurediethylester, Maleinsäurediethylester, Maleinsäuredimethylester, Maleinsäuredibutylester, Acrylnitril, Methacrylnitril, Vinylacetat und Vinylpropionat. Pro 100 Gew.-Teile der Monomeren der Gruppe a) setzt man bei der Copolymerisation 0 bis 20, vorzugsweise 0,5 bis 5 Gew.-Teile der Monomeren der Gruppe c) ein.

Als Vernetzer verwendet man eine Verbindung, die mindestens zwei ethylenisch ungesättigte Doppelbindungen enthält. Geeignete Vernetzer sind beispielsweise N,N'-Methylen-bisacrylamid, Polyethylenglykoldiacrylate und Polyethylenglykoldimethacrylate, die sich jeweils von Polyethylenglykolen eines Molekulargewichts von 126 bis 8500 ableiten, Trimethylolpropantriacrylat, Trimethylolpropantrimethacrylat, Butandioldiacrylat, Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte Anlagerungsprodukte von Ethylenoxid und/oder Propylenoxid an Trimethylolpropan, mindestens zweifach mit Acrylsäure oder Methacrylsäure verestertes Glycerin oder Pentaerythrit, Triallylamin, Tetraallylethylendiamin, Divinylbenzol, Diallylphthalat, Polyethylenglykoldivinylether, Trimethylolpropandiallylether, Polyethylenglykoldivinylether, Butandioldivinylether, Polyethylenglykoldiallylether, Butandioldiallylether und Divinylethylenharnstoff. Die Vernetzer werden, bezogen auf 100 Gew.-Teile der Monomeren der Gruppen a) bis c), in einer Menge von 0,01 bis 5, vorzugsweise 0,1 bis 3 Gew.-Teilen bei der Copolymerisation verwendet.

Die Monomeren werden in wäßriger Lösung polymerisiert. Die wasserunlöslichen Monomeren, die gegebenenfalls mitverwendet werden können, werden üblicherweise mit Hilfe von Emulgatoren in der wäßrigen Lösung fein verteilt. Geeignete Emulgatoren sind beispielsweise ethoxylierte Nonylphenole, ethoxyliertes Rizinusöl, Alkylsulfate, Sorbitanfettsäureester, ethoxylierte Sorbite, ethoxylierte Sorbitanfettsäureester und Alkylsulfonate. Die Emulgatoren werden in einer Menge von 0 bis 3 Gew.-Teilen pro 100 Gew.-Teile der Monomeren der Gruppen a) bis c) eingesetzt. Die Konzentration der wäßrigen Monomerlösung liegt vorzugsweise in dem Bereich von 30 bis 50 Gew.%.

Als Initiatoren eignen sich hauptsächlich wasserlösliche Radikale bildende Verbindungen, z.B. Azostarter wie 2,2'-Azobis-(N,N'-dimethylenisobutyramidin)-dihydrochlorid, 2,2'-Azobis-(2-amidinopropan)-dihydrochlorid, 2,2'-Azobis-(N,N'-dimethylenisobutyramidin), 4,4'-Azobis-(4-cyanopentancarbonsäure), 2-Carbamoylazo-isobutyronitril sowie Dibenzoylperoxid, Dilaurylperoxid, Di-2-ethylhexylperoxidicarbonat, Dicyclohexylperoxidicarbonat, Bis-(4-tert.-butylcyclohexyl)-peroxidicarbonat, tert.-Butylperpivalat, tert.-Butylperbenzoat, tert.-Butylpermaleinat, Di-tert.-butylperoxid, tert.-Butylhydroperoxid, Wasserstoffperoxid, Ammoniumpersulfat, Kaliumpersulfat, Natriumpersulfat sowie Redoxkatalysatoren, wobei als reduzierende Komponente Eisen(II)-ammoniumsulfat, Ascorbinsäure, Natriumhydroximethansulfinat, Dinatriumdisulfit und Natriumhydrogensulfit in Betracht kommen. Die Initiatoren können entweder allein oder in Mischung angewendet werden. Die Zerfallsgeschwindigkeit der sehr hoch zerfallenden Peroxide kann durch Mitverwendung von organischen Metallkomplexverbindungen, z.B. Kupferacetylacetonat, erniedrigt werden, so daß die Zerfallsgeschwindigkeit der Peroxide der jeweils gewählten Polymerisationstemperatur angepaßt werden kann. Vorzugsweise verwendet man Redoxkatalysatoren aus einem oder mehreren Peroxiden und einem Reduktionsmittel. Besonders bevorzugt ist der Einsatz von Persulfaten oder Perestern oder Mischungen aus Persulfaten und Perestern als ein Bestandteil von Redox-Polymerisationsinitiatoren. Die Polymerisationsinitiatoren werden in einer Menge von 0,01 bis 5, vorzugsweise 0,2 bis 3 Gew.%, bezogen auf die bei der Polymerisation eingesetzten Monomeren angewendet.

Um das Molekulargewicht der Polymerisate zu regeln, kann man auch in Gegenwart eines Polymerisationsreglers polymerisieren, z.B. von Mercaptoethanol, Mercaptopropanol, Thioglykolsäure, Dodecylmercaptan, Ameisensäure oder halogenierten Kohlenwasserstoffen wie Brommethan oder Tetrachlorkohlenstoff. Die Polymerisationsregler werden in einer Menge von 0 bis 3 Gew.%, bezogen auf die an der Polymerisation eingesetzten Monomeren, verwendet.

Die Copolymerisation der wäßrigen Lösung der Monomeren wird erfindungsgemäß mehrstufig in einer diskontinuierlich arbeitenden Mischvorrichtung unter ständiger Durchmischung der Stoffe in allen Stufen durchgeführt. Da die wäßrige Monomerlösung im Verlauf der Polymerisation über eine gelartige Masse zu einem feinteiligen Polymerisat übergeht, ist es erforderlich, in allen Verfahrensstufen für eine kräftige Durchmischung zu sorgen. Als diskontinuierlich arbeitende Mischvorrichtungen kommen beispielsweise Kneter in Betracht, die einen Selbstreinigungseffekt von mindestens 80% aufweisen. Die Kneter können eine oder mehrere Wellen aufweisen. Die Copolymerisation kann auch vorteilhaft in einem einwelligen zylindrischen Mischer durchgeführt werden, an dessen Rührwelle Scheibensegmente angeordnet sind, die am äußeren Ende Mischbarren in einer solchen Anordnung aufweisen, daß dadurch eine intensive zirkulierende Durchmischung der dem Mischer zugegebenen Stoffe bewirkt wird. Der einwellige zylindrische Mischer hat ein Verhältnis von Länge:Durchmesser von 2:1 bis 20:1. Auf der Rührwelle sind die Scheibensegmente propellerartig angeordnet. Auf die gesamte Länge der Rührwelle verteilt befinden sich 2 bis 25 dieser Scheibensegmente, wobei ein Scheibensegment aus 2 bis 7 Einzelelementen besteht, die propellerartig angeordnet sind. Die Mischelemente, die sich am äußeren Ende der Scheibensegmente befinden, bewirken die Förderung der im Stadium der Polymerisation befindlichen Mischung innerhalb des Mischgeräts und verhindern gleichzeitig, daß sich Polymergel an der Innenwand des Mischers ablagert, weil die Mischelemente in der Nähe der inneren Wandung des zylinderförmigen Mischers vorbeilaufen. Als Mischelemente sind z.B. wandgängige Mischbarren oder pflugscharartig ausgeformte Aufsätze geeignet. In dem Mischer befinden sich außer dem noch eingebaute Gegenhaken mit Flansch um das bei der Polymerisation entstehende Gel von den Scheibensegmenten der Rührwelle und den Mischbarren zu entfernen. Auch derartige Mischvorrichtungen verfügen über einen Selbstreinigungseffekt, der oberhalb von 80 % liegt. Geeignete Vorrichtungen, die einen hohen Selbstreinigungseffekt haben, sind bekannt und werden beispielsweise in Chem.-Ing.-Techn. 57, 1005 (1985) beschrieben.

Die für die Polymerisation geeigneten Vorrichtungen werden mit der wäßrigen Monomerlösung, die einen Initiator gelöst oder dispergiert enthalten kann, beschickt. Der Füllgrad der in Betracht kommenden Polymerisationsvorrichtungen beträgt bis zu 45 % und liegt vorzugsweise zwischen 20 und 35 Vol.%. Die Polymerisationsvorrichtungen können nach Bedarf beheizt oder gekühlt werden. Sie sind für das Arbeiten unter vermindertem sowie unter erhöhtem Druck ausgelegt.

In der ersten Stufe der Copolymerisation wird die wäßrige Monomerlösung in Gegenwart von Polymerisationsinitiatoren bei Temperaturen von 45 bis 95°C und einem Druck von 0,1 bis 0,8 bar unter partiellem Abdestillieren von Wasser copolymerisiert. Das verdampfte Wasser wird über Druckhaltevorrichtungen aus dem Mischer entfernt. Durch das Abdestillieren des Wassers unter vermindertem Druck ist es möglich, die Polymerisationstemperatur exakt einzustellen und die Polymerisationswärme auf diese Weise aus dem System zu entfernen. In der ersten Stufe der Polymerisation wird soviel Wasser abdestilliert, daß etwa 30 bis 90 gew.%ige Polymergele vorliegen, die bereits am Ende der ersten Stufe in ein krümeliges Gel übergehen können. Die Polymerisationsdauer in der 1. Stufe beträgt zwischen etwa 5 und 60 Minuten.

In der zweiten Stufe der Copolymerisation wird die Temperatur des Reaktionsgemisches auf 100 bis 170, vorzugsweise 120 bis 140°C erhöht. Das bedeutet, daß der Druck in der Vorrichtung aufgrund des Wassergehalts der polymerisierenden Mischung auf Werte in dem Bereich von 1 bis 8, vorzugsweise 2 bis 5 bar ansteigt. Die Polymerisation wird in der zweiten Stufe vervollständigt. Auch in dieser Stufe des Verfahrens muß das Reaktionsgemisch durchgemischt bzw. geschert werden. Die thermische Behandlung des Copolymerisats in der zweiten Stufe dauert etwa 10 bis 100, vorzugsweise 15 bis 50 Minuten. Nach dieser Behandlung erfolgt der Druckausgleich mit der Atmosphäre.

In der dritten Stufe der Copolymerisation wird - ebenfalls unter ständiger Durchmischung der krümelig gewordenen Reaktionsmasse - der Wassergehalt des so hergestellten feinteiligen Copolymerisats bei 70 bis 180°C unter vermindertem Druck auf Werte von 0,5 bis 10, vorzugsweise 3 bis 6 Gew.% erniedrigt. Der Druck, bei der die Trocknung der Copolymerisate vorgenommen wird, ist selbstverständlich temperaturabhängig und liegt in der Größenordnung von 10 bis 800 mbar. Die Dauer der Trocknung beträgt etwa 10 bis 100 Minuten. Das vernetzte Copolymerisat kann jedoch auch bei Normaldruck und Temperaturen zwischen 120 und 180°C getrocknet werden.

Man erhält auf diese Weise ein rieselfähiges feinteiliges Polymerisat, das eine sehr hohe Wasseraufnahme hat und beispielsweise als Bodenverbesserungsmittel bzw. als Absorptionsmittel in Hygieneartikeln, z.B. Windeln, verwendet wird. Die in den Beispielen angegebenen Teile sind Gewichtsteile, die Angaben in Prozent beziehen sich auf das Gewicht der Stoffe, sofern nichts anderes angegeben ist.

Bestimmung des Absorptionsvermögens der Polymerisate

In den Beispielen wurde jeweils das Absorptionsvermögen des hergestellten Polymerisats für physiologische Kochsalzlösung angegeben. Es wurde dadurch ermittelt, daß man jeweils 0,2 g des Polymeren in einem teebeutelartig geformten Filterpapiertütchen einschloß und 10 min lang in eine 0,9 %ige wäßrige Kochsalzlösung eintauchte. Nach Subtraktion der Sorption des leeren Tütchens errechnete man jeweils die Aufnahmefähigkeit des Polymerisats.

Bestimmung der löslichen Anteile

Der Gehalt der nicht in das Netzwerk der Polymerisate eingebundenen löslichen Anteile wurde durch 8stündige Quellung des Polymeren in Wasser und Messung des Kohlenstoffgehalts der wäßrigen Lösung ermittelt.

Beispiel 1

In einem 6 l fassenden einwelligen zylindrischen Mischer, an dessen Rührwelle Scheibensegmente angeordnet sind, die am äußeren Ende Mischbarren in einer solchen Anordnung aufweisen, daß dadurch eine intensive zirkulierende Durchmischung des Inhalts bewirkt wird, wird eine Monomerlösung, deren Temperatur 20°C beträgt und die aus 500 Teilen Acrylsäure, 6 Teilen N,N'-Methylen-bisacrylamid und 120 Teilen Wasser besteht, unter Stickstoff vorgelegt, so daß der Mischer zu 35 % gefüllt ist. Der Mischer hat ein Verhältnis von Länge:Durchmesser von 7:1 und ist für das Arbeiten unter vermindertem sowie unter erhöhtem Druck ausgelegt. Auf der Rührwelle des Mischers sind jeweils 8 Scheibensegmente in einem Abstand von 15 cm angeordnet, wobei ein Scheibensegment aus drei Einzelelementen besteht. Unter ständiger Durchmischung der Monomerlösung werden dann 150 Teile 50 %ige wäßrige Natronlauge zugesetzt, um die Acrylsäure zu 27 % zu neutralisieren. Während der Neutralisation wird das Reaktionsgemisch auf 30°C gekühlt. Danach werden 5 Teile Ammoniumperoxidisulfat in 25 Teile Wasser eingemischt. Rührwelle und Gehäuse des einwelligen Mischers sind beheizbar und werden auf eine Temperatur von 45°C und der Druck im Innern des Mischers auf 200 mbar eingestellt. Die Polymerisationsreaktion wird dann durch Zugabe von 1 Teil Natriumhydrogensulfit in 10 Teilen Wasser gestartet. Gleichzeitig fügt man innerhalb von 10 min 300 Teile einer 50 %igen wäßrigen Kalilauge zu. Die Temperatur des Reaktionsgemisches steigt auf 72°C an. Während der Polymerisation werden 270 Teile Wasser abdestilliert. Die Polymerisation in der ersten Stufe dauert 25 min. In der zweiten Stufe der Polymerisation wird zunächst der Druckausgleich unter einer Stickstoffatmosphäre bis auf 1 bar vorgenommen, der Mischer dann druckdicht verschlossen und das Reaktionsgemisch darin auf eine Temperatur von 135°C erhitzt. Dadurch steigt der Druck im Mischer auf 4 bar. Das Reaktionsgemisch wird 20 min bei dieser Temperatur unter ständigem Durchmischen unter Druck behandelt. Nach dem Druckausgleich mit der Atmosphäre wird in der dritten Stufe der Wassergehalt des so hergestellten Copolymerisats bei einem Druck von 50 mbar und einer Temperatur von 140°C unter ständigem Durchmischen auf 2,8 % erniedrigt. Danach erfolgt der Druckausgleich mit der Atmosphäre. Anschließend kann dem Mischer ein vernetztes, feinteiliges wasserabsorbierendes Polymerisat entnommen werden. Der mittlere Teilchendurchmesser des Polymerisats beträgt 3 mm. 1 g des Polymerisats nimmt 61 g physiologische Kochsalzlösung auf. Durch Quellung in Wasser lassen sich ca. 9 % lösliche Anteile extrahieren.

Beispiel 2

In dem in Beispiel 1 beschriebenen Mischer wird bis zu einem Füllungsgrad von 30 % unter Stickstoff und fortwährendem Rühren eine Lösung aus 300 Teilen Acrylsäure, 100 Teilen Acrylamid, 35 Teilen N-Vinylpyrrolidon und 5 Teilen Butandioldivinylether in 150 Teilen Wasser mit 220 Teilen 50 %iger wäßriger Kalilauge teilneutralisiert, wobei die Temperatur durch Kühlung auf 30°C begrenzt wird. Nach Einmischen einer Lösung von 5 Teilen Kaliumperoxidisulfat in 150 Teilen Wasser wird die Rührwelle und das Gehäuse des einwelligen Mischers auf 45°C aufgeheizt und im Inneren des Mischers ein Druck von 400 bar eingestellt. Die Polymerisation wird durch Zufügen von 0,5 Teilen Natrium-hydroximethansulfinat in 5 Teilen Wasser gestartet. Während der 15minütigen Polymerisation werden 120 g 50 %ige wäßrige Kalilauge zugegeben und 260 Teile Wasser abgezogen. Die Temperatur des Reaktionsgemisches steigt bis auf 83°C. In der zweiten Stufe wird nach Druckausgleich mit Stickstoff auf 1 bar und dichtem Verschließen des Mischers das Reaktionsgemisch auf 120°C aufgeheizt, wobei der Druck im Mischer auf ca. 3 bar steigt. Das Reaktionsgemisch wird 20 min. unter diesen Bedingungen unter ständigem Rühren nachbehandelt. Schließlich wird in der dritten Stufe nach durchgeführtem Druckausgleich auf 30 mbar evakuiert und das so hergestellte Copolymerisat bei 150°C unter intensivem Durchmischen zu einem feinkrümmeligen Material getrocknet. Nach Belüften des Mischers kann ein vernetztes, feinteiliges wasserabsorbierendes Polymerisat mit einem mittleren Teilchendurchmesser von 3 mm entnommen werden. 1 g des Polymerisats sorbiert 54 g physiologische Kochsalzlösung. Durch Quellung in Wasser können ca. 7 % lösliche Anteile abgetrennt werden.

Beispiel 3

In dem in Beispiel 1 beschriebenen einwelligen Mischer wird bis zu einem Füllungsgrad von 40 % unter Stickstoff und Rühren eine Lösung aus 250 Teilen Acrylsäure, 40 Teilen Methacrylsäure, 43 Teilen Itaconsäure, 7 Teilen Divinylbenzol und 7 Teilen Natrium-pentadecylsulfonat in 250 Teilen Wasser mit 230 Teilen 50 %iger wäßriger Kalilauge teilneutralisiert und währenddessen die Temperatur der Lösung durch Wärmeabfuhr auf 30°C gehalten. Nach Zufügen einer Lösung von 10 Teilen Natriumperoxidisulfat in 50 Teilen Wasser werden Rührwelle und Gehäuse des einwelligen Mischers auf 45°C aufgeheizt, der Mischer auf 300 mbar evakuiert und die Polymerisation durch Zugabe von 0,5 Teilen Ascorbinsäure in 30 Teilen Wasser gestartet. Das Reaktionsgemisch erreicht während der 25minütigen Polymerisation in dieser ersten Stufe eine Temperatur von 72°C. Es werden 230 Teile Wasser abdestilliert. Für die zweite Stufe der Polymerisation wird der Druckausgleich gegen eine Stickstoffatmosphäre bis auf 1 bar durchgeführt, der Mischer druckdicht verschlossen und das Reaktionsgemisch auf 130°C aufgeheizt, wobei ein Druck von ca. 3,5 bar aufgebaut wird. Man beläßt 35 min. bei dieser Temperatur und diesem Druck unter Durchmischen zur Nachbehandlung. Im Anschluß daran wird die Temperatur auf 60°C gesenkt, der Druck bis auf Atmosphärendruck abgebaut und mit 120 Teilen 50 %iger wäßriger Kalilauge unter intensivem Durchmischen des Polymerisats nachneutralisiert. In der dritten Stufe wird auf 20 mbar evakuiert und bei 120°C bis auf einen Restwassergehalt von 5,2 % zu einem feinteiligen Feststoff mit einem mittleren Teilchendurchmesser von 3 mm getrocknet.

1 g des so gewonnenen Copolymeren nimmt 58 g physiologische Kochsalzlösung auf. Durch Quellung in Wasser lassen sich ca. 7 % lösliche Anteile extrahieren.

## Patentansprüche

1. Verfahren zur diskontinuierlichen Herstellung von vernetzten, feinteiligen, wasserabsorbierenden Polymerisaten durch Copolymerisieren von 100 Gew.-Teilen eines Monomeren aus der Gruppe a) oder eines Monomerengemisches aus den Gruppen

a) 50 bis 100 Gew.-Teilen zu jeweils 0 bis 100 Mol% mit Alkalimetall- oder Ammoniumbasen neutralisierter Acrylsäure oder Methacrylsäure, Acrylamid, Methacrylamid und N-Vinylpyrrolidon,
b) 0 bis 30 Gew.-Teilen anderen wasserlöslichen monoethylenisch ungesättigten Monomeren und
c) 0 bis 20 Gew.-Teilen wasserunlöslichen monoethylenisch ungesättigten Monomeren

mit 0,01 bis 5 Gew.-Teilen eines mindestens zwei ethylenisch ungesättigte Doppelbindungen enthaltenden Monomeren als Vernetzer in 20 bis 80 gew.%iger wäßriger Lösung in Gegenwart von Initiatoren bei Temperaturen oberhalb von 45°C, dadurch gekennzeichnet, daß man die Copolymerisation mehrstufig in einer diskontinuierlich arbeitenden Mischvorrichtung unter ständiger Durchmischung in allen Stufen durchführt, wobei man

in der 1. Stufe die wäßrige Monomerlösung bei Temperaturen von 45 bis 95°C und einem Druck von 0,1 bis 0,8 bar unter partiellem Abdestillieren von Wasser copolymerisiert,
in der 2. Stufe die Copolymerisation bei Temperaturen von 100 bis 170°C unter einem Druck bis zu 8 bar vervollständigt und nach dem Entspannen
in der 3. Stufe den Wassergehalt des so hergestellten feinteiligen Copolymerisats unter vermindertem Druck bei 70 bis 180°C oder unter Atmosphärendruck bei 120 bis 180°C auf 0,5 bis 10 Gew.% erniedrigt.

2. Verfahren nach Anspruch 1, dadurch gekennzeichnet, daß als diskontinuierlich arbeitende Mischvorrichtung ein Kneter mit einem Selbstreinigungseffekt von mindestens 80 % eingesetzt wird.

3. Verfahren nach den Ansprüchen 1 und 2, dadurch gekennzeichnet, daß man Acrylsäure, die zu 10 bis 100 Mol.% mit Natronlauge und/oder Kalilauge neutralisiert ist, mit einem mindestens zwei ethylenisch ungesättigte Doppelbindungen enthaltenden Monomeren copolymerisiert.

4. Verfahren nach Anspruch 1, dadurch gekennzeichnet, daß man als Vernetzer N,N'-Methylen-bisacrylamid, Polyethylenglykoldiacrylate, Polyethylenglykoldivinylether, Butandioldivinylether, Trimethylolpropantriacrylat, Butandioldiacrylat, Polyethylenglykoldiallylether und/oder Butandioldiallylether einsetzt.

## Claims

1. A process for the batchwise preparation of a crosslinked, finely divided, water-absorbing polymer by copolymerizing 100 parts by weight of a monomer from group a) or of a monomer mixture from groups

a) 50–100 parts by weight of acrylic acid or methacrylic acid, each of which has been neutralized with from 0 to 100 mol% of an alkali metal or ammonium base, acrylamide, methacrylamide and N-vinylpyrrolidone,
b) 0–30 parts by weight of other water-soluble monoethylenically unsaturated monomers and
c) 0–20 parts by weight of water-insoluble monoethylenically unsaturated monomers

with from 0.01 to 5 parts by weight of, as a crosslinking agent, a monomer containing two or more ethylenically unsaturated double bonds, in 20–80% strength by weight aqueous solution in the presence of an initiator at above 45°C, wherein the copolymerization is carried out as a multistage process in a batchwise mixing apparatus, with constant thorough mixing in all stages, in the first stage the aqueous monomer solution being copolymerized at from 45 to 95°C under from 0.1 to 0.8 bar with removal of some of the water by distillation, in the second stage the copolymerization being completed at from 100 to 170°C under a pressure up to 8 bar and, in the third stage, after the pressure has been let down, the water content of the resulting finely divided copolymer being reduced to 0.5–10% by weight under reduced pressure at from 70 to 180°C or under atmospheric pressure at from 120 to 180°C.

2. A process as claimed in claim 1, wherein the batchwise mixing apparatus used is a kneader having a self-purging effect of not less than 80%.

3. A process as claimed in either of claims 1 and 2, wherein acrylicacid, which has been neutralized with from 10 to 100 mol% of sodium hydroxide solution and/or potassium hydroxide solution, is copolymerized with a monomer containing two or more ethylenically unsaturated double bonds.

4. A process as claimed in claim 1, wherein N, N'-methylenebisacrylamide, polyethylene glycol diacrylates, polyethylene glycol divinyl ether, butanediol divinyl ether, trimethylolpropane triacrylate, butanediol diacrylate, polyethylene glycol diallyl ether and/or butanediol diallyl ether are used as crosslinking agents.

## Revendications

1. Procédé de préparation discontinue de polymères réticulés, en fines particules et absorbant l'eau, par copolymérisation de 100 parties en poids d'un monomère du groupe a) ou d'un mélange de monomères des groupes

a) 50 à 100 parties en poids d'acide acrylique ou d'acide méthacrylique neutralisés à raison de 0 à 100 moles%, suivant le cas avec des bases de métaux alcalins ou d'ammonium, d'acrylamide, de méthacrylamide et de N-vinylpyrrolidone,
b) 0 à 30 parties en poids d'autres monomères monoéthyléniquement insaturés solubles dans l'eau et
c) 0 à 20 parties en poids de monomères monoéthyléniquement insaturés insolubles dans l'eau

avec 0,01 à 5 parties en poids d'un monomère contenant au moins deux doubles liaisons éthyléniquement insaturées comme agent de réticulation, en solution aqueuse à 20 à 80% en poids, en présence d'initiateurs et à des températures supérieures à 45°C, caractérisé en ce que l'on conduit la copolymérisation en plusieurs étapes, dans un dispositif de mélange travaillant en discontinu et en malaxant continuellement pendant chaque étape, procédé suivant lequel dans la première étape, on copolymérise la solution aqueuse de monomères à des températures de 45 à 95°C et sous une pression de 0,1 à 0,8 bar avec séparation partielle de l'eau par distillation, dans la seconde étape, on termine la copolymérisation à des températures de 100 à 170°C et sous une pression pouvant atteindre 8 bars et, après la détente, dans la troisième étape, on réduit la teneur en eau du copolymère en fines particules ainsi préparé à une teneur de 0,5 à 10% en poids, sous pression réduite à une température de 70 à 180°C, ou à la pression atmosphérique à une température de 120 à 180°C.

2. Procédé suivant la revendication 1, caractérisé en ce que l'on met en œuvre, comme dispositif mélangeur travaillant en discontinu, un malaxeur ayant un effet d'autonettoyage d'au moins 80%.

3. Procédé suivant les revendications 1 et 2, caractérisé en ce que l'on copolymérise de l'acide acrylique, qui est neutralisé à raison de 10 à 100 moles% avec de la lessive de soude et/ou de la lessive de potasse, avec un monomère contenant au moins deux doubles liaisons éthyléniquement insaturées.

4. Procédé suivant la revendication 1, caractérisé en ce que l'on met en œuvre, comme agents de réticulation, le N,N'-méthylène-bisacrylamide, le diacrylate de polyéthylèneglycol, le polyéthylèneglycoldivinyléther, le butanedioldivinyléther, le triacrylate de triméthylolpropane, le diacrylate de butanediol, le polyéthylèneglycoldiallyléther et/ou le butanedioldiallyléther.